# FASCICULE DE BREVET EUROPEEN

(11) **EP 3 728 182 B1**
(45) Date de publication et mention de la délivrance du brevet: **17.04.2024**
(21) Numéro de dépôt: 18833922.0
(22) Date de dépôt: 12.12.2018
(51) Int. Cl.: C07C 233/47, A61K 8/34, A61K 8/44, C07C 31/20, A61K 8/49, A61Q 19/00, A61Q 19/06

(54) **NOUVELLE COMPOSITION DE LIPOAMINOACIDES ET DE DIOLS, LE PROCÉDÉ POUR LEUR PRÉPARATION ET COMPOSITION COSMÉTIQUE OU PHARMACEUTIQUE EN RÉSULTANT**
NEUE ZUSAMMENSETZUNG AUS LIPOAMINOSÄUREN UND DIOLEN, VERFAHREN ZU IHRER HERSTELLUNG UND DARAUS RESULTIERENDE KOSMETISCHE ODER PHARMAZEUTISCHE ZUSAMMENSETZUNG
NEW COMPOSITION OF LIPOAMINO ACIDS AND DIOLS, PROCESS FOR THE PREPARATION THEREOF, AND COSMETIC OR PHARMACEUTICAL COMPOSITION RESULTING THEREFROM

(30) Priorité: 20.12.2017 FR 1762585
(43) Date de publication de la demande: 28.10.2020
(73) Titulaire: Société d'Exploitation de Produits pour les Industries Chimiques SEPPIC, 75321 Paris cedex 07 (FR)
(72) Inventeur: GUILBOT, Jérôme, 81100 Castres (FR); DACOSTA, Georges, 81710 Saix (FR); BARTHE, Virginie, 75321 Paris Cedex 07 (FR)
(74) Mandataire: Air Liquide
(86) Numéro de dépôt international: PCT/FR2018/053213
(87) Numéro de publication internationale: WO 2019/122601

(56) Documents cités:
- WO-A1-2017/153161
- FR-A1- 2 765 105
- FR-A1- 2 771 632
- US-A1- 2013 101 530
- PROSERPIO G ET AL: "SOSTANZE NO CONSERVANTI IN GRADO DI INIBIRE LA CRESCITA MICROBICA NEI COSMETICI", COSMETICS AND TOILETRIES. ED. ITALIANA, COSMEO EDITRICE, MILANO, IT, vol. 17, no. 3, 1 janvier 1996 (1996-01-01), pages 11-13,16, XP002074040, ISSN: 1121-1377

## Description

La présente invention concerne un nouveau procédé de préparation de dérivés N-acylés d'acides aminés, d'(oligo)peptides ou bien d'hydrolysats partiels ou totaux de protéines, présentant un taux de conversion élevé des matières premières mises en oeuvre.

Les dérivés N-acylés d'acides aminés ou également appelés lipoaminoacides (LAA), sont des agents tensioactifs anioniques, qui sont constitués par une tête polaire provenant d'un résidu d'au moins un acide aminé, ou bien d'un résidu d'(oligo)-peptides ou bien de résidus d'hydrolysats partiels ou totaux de protéines, et par une chaîne hydrocarbonée de nature lipophile, provenant de chlorures d'acides gras ou d'ester méthyliques d'acides gras, eux-mêmes issus de la filière oléochimique.

Ces dérivés N-acylés d'acides aminés, d'(oligo)-peptides ou bien d'hydrolysats partiels ou totaux de protéines, sont communément utilisés tout d'abord comme ingrédient apportant des propriétés moussantes et nettoyantes pour la préparation de compositions cosmétiques, comme par exemple des gels douche ou des shampoings, ou bien comme ingrédients apportant des propriétés biologiques pour la préparation de compositions cosmétiques destinées à prévenir ou corriger les effets inesthétiques de la peau ; lesdites propriétés biologiques sont par exemple des propriétés anti-âges, amincissantes, raffermissantes, dépigmentantes, propigmentantes.

Les dérivés N-acylés d'acides aminés, d'(oligo)-peptides ou bien d'hydrolysats partiels ou totaux de protéines sont communément synthétisés par acylation d'un ou plusieurs acides aminés en présence de chlorure d'acide, dans les conditions expérimentales dites de Schotten-Baumann.

Un tel procédé est par exemple divulgué dans les brevets américains US 2,463,779 et US 6,703,517, dans la publication J. Am. Oil Chem. Soc.- 78 (1956) 172, et dans les demandes internationales publiées sous les numéros WO 92/21318 et WO 94/26694.

Ce procédé d'acylation comprend une étape préalable de salification de l'acide aminé, suivie d'une étape d'acylation du sel d'aminoacide avec un chlorure d'acide, puis de l'acidification du sel N-acylé obtenu.

La première étape consiste à neutraliser l'acide aminé, solubilisé préalablement dans de l'eau ou dans un mélange d'eau et d'un cosolvant organique, par une base minérale, le plus souvent de la soude ou de la potasse aqueuse. Le groupement carboxyle se retrouve alors sous une forme ionisée, permettant ainsi une meilleure solubilité de l'acide aminé dans l'eau. Le pH de cette solution aqueuse se situe entre 9,0 et 12,0, ce qui permet de s'assurer que la fonction amine de l'acide aminé n'est pas protonée.

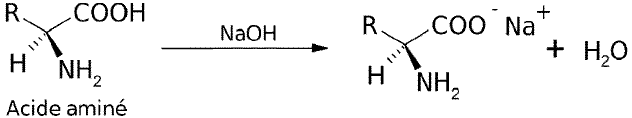

La seconde étape est l'étape d'acylation à proprement dite. A ce stade, le chlorure d'acide est ajouté progressivement à la solution neutralisée d'acide aminé, à température ambiante. La fonction amine nucléophile attaque le carbone électrophile de la fonction carbonyle. Il en résulte la formation d'une liaison amide entre les deux substrats de départ ainsi que la formation d'acide chlorhydrique. Cet acide est directement neutralisé *in situ* par ajout progressif d'une base minérale (régulation du pH autour de 10,0).

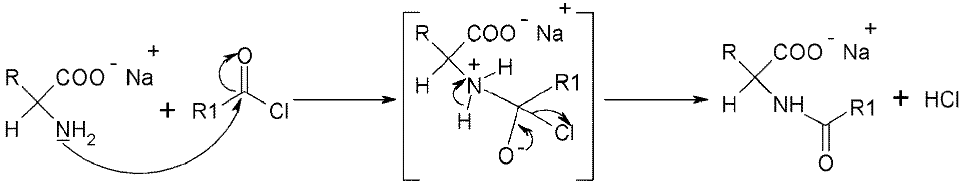

A ce stade, la réaction secondaire d'hydrolyse du chlorure d'acide en savon est également possible. Elle doit cependant être minimisée de façon à atteindre une conversion satisfaisante de l'acide aminé en son dérivé N-acylé et à les isoler efficacement, car une teneur trop élevée en savon (ou sel d'acide gras) peut induire un déphasage du milieu réactionnel et/ou des problèmes d'odeur ou de toxicité (pour des chaînes acyles en C₈ et en C'₁₁ par exemple).

Les deux principaux paramètres réactionnels qui permettent de contrôler la formation de savon sont la vitesse d'agitation lors de la phase réactionnelle, dont l'optimisation permet une amélioration de la surface de contact du chlorure avec le milieu, et l'ajout éventuel, lors de l'étape de solubilisation de l'acide aminé, d'un cosolvant d'acylation, comme par exemple l'acétone, la méthyl éthyl cétone, l'isopropanol, ou des glycols.

Cet ajout de cosolvant permet d'améliorer l'affinité du chlorure d'acide avec le milieu réactionnel. Dans un tel cas, le cosolvant d'acylation doit être choisi judicieusement de façon à éviter ou minimiser la formation de nouveaux produits secondaires issus de la réaction entre ce même cosolvant et le chlorure d'acide, comme par exemple des sous-produits provenant de réactions secondaires d'estérification.

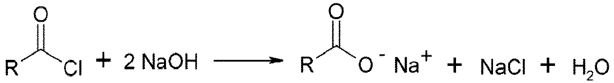

La dernière étape consiste en une étape de finition, de mise en forme du dérivé N-acylé formé, et deux alternatives sont possibles.

La première consiste à régler la valeur du pH du milieu réactionnel obtenu autour de 7. Le dérivé N-acylé est isolé tel quel en solution, sans aucune purification supplémentaire, et comprend les sels d'acylation, les acides aminés non réagis, de l'éventuel cosolvant). Il se présente ainsi sous une forme salifiée, et plus précisément une forme carboxylate, en solution aqueuse et avec une pureté généralement inférieure à 50%. 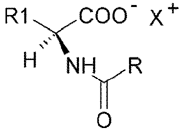
R1 = Chaîne latérale de l'acide aminé,
R = Chaîne cocoyle,
X = Contre-ion.

La seconde consiste à faire précipiter le dérivé N-acylé en acidifiant le mélange réactionnel à une valeur de pH voisine de 2, puis à réaliser plusieurs opérations de filtration et de lavage, se concluant par un séchage final du milieu obtenu. Cette procédure permet ainsi d'éliminer tous les sels générés au cours de la réaction d'acylation, l'éventuel cosolvant d'acylation et tous les acides aminés non réagis. Dans ce cas, le dérivé N-acylé se présente sous une forme non salifiée, avec une fonction acide carboxylique, et une forme solide, plus particulièrement pulvérulente, et avec 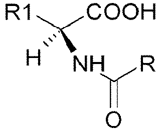
R1 = Chaîne latérale de l'acide aminé,
R = Chaîne cocoyle,
une pureté supérieure à 80%. X = Contre-ion.

Le procédé Schotten-Baumann précédemment décrit, présente comme avantage de conduire des réactions de N-acylation avec une cinétique rapide, du fait de la réactivité très élevée des chlorures d'acide vis-à-vis des composés et fonctions nucléophiles (par exemple la fonction amine), sans apport important d'énergie comme par exemple l'énergie thermique, dans un milieu solvant majoritairement constitué d'eau, avec des rendements élevés.

Cependant le formulateur de produits cosmétiques rencontre aujourd'hui des difficultés quant à la mise en oeuvre de poudres, lorsqu'il s'agit de préparer des produits finis. D'autre part, les acides gras résiduels, dont le radical acyle comporte moins de 12 atomes de carbone, peuvent générer, lorsqu'ils sont présents en quantité trop importante, des réactions d'irritations cutanés et/ou des odeurs incommodantes, incompatibles avec une utilisation dans la préparation de compositions cosmétiques. C'est la raison pour laquelle il existe un besoin de développer une méthode de synthèse directe de sels d'aminoacides N-acylés salifiés liquides et de haute pureté sans étapes de précipitation / filtration / séchage.

Les inventeurs ont trouvé qu'il était possible d'atteindre ce résultat en utilisant des cosolvants d'acylation pouvant également être utilisés comme solvants finaux de dilution, étant entendu que de tels cosolvants doivent aussi permettre de contrôler et/ou de minimiser la formation d'acides gras lors de l'étape d'acylation, être peu réactifs vis-à-vis des chlorures d'acides dans les conditions de réactions, permettre l'élimination efficace des sels générés au cours de l'acylation par simple décantation liquide/liquide à chaud et enfin permettre d'obtenir une solution d'aminoacides N-acylés homogènes à pH neutre.

La demande de brevet français publiée sous le numéro de publication FR 2765105A2 décrit un procédé de préparation d'un mélange de dérivés N-acylés d'acides aminés mettant notamment en oeuvre une quantité non définie de propylène glycol (ou 1,2-propanediol), du chlorure de lauroyle, et indique un taux d'acide laurique résiduel dans le produit final de 15% massique.

C'est pourquoi, selon un premier aspect, l'invention a pour objet le procédé tel que défini à la revendication 1.

Selon un aspect particulier, l'invention a pour objet le procédé tel que défini ci-dessus, de préparation de la composition (C₁), pour laquelle dans la formule (I), le radical divalent Y représente le radical divalent de formule (IIₐ₁) :

-[NH-CH(R₂)-C(=O)-]ₘ- (IIₐ₁),

dans laquelle m représente un nombre entier supérieur ou égal à un et inférieur ou égal à quatre et R₂ représente un atome d'hydrogène ou un radical choisi parmi les radicaux méthyle, isopropyle, isobutyle, 1-méthyl propyle, benzyle ou 3-amino propyle ; par exemple le radical divalent de formule (II'ₐ₁) :

-NH-CH₂-C(=O)- (II'ₐ₁),

comme la composition (C₁) telle que définie précédemment, caractérisée en ce que dans les formules (I) et (V), le radical monovalent X-(CH₂)_{P}-C(=O)- représente le radical octanoyle.

Selon un autre aspect particulier, la composition (C₁) telle que définie ci-dessus est caractérisée en ce que dans les formules (I) et (V), le radical monovalent X-(CH₂)ₚ-C(=O)-représente le radical 10-undécylénoyle et Y représente le radical divalent de formule (IIₐ₂) :

-[NH-CH(Benzyl)-C(=O)-]ₘ- (IIₐ₂).

Par diol comportant de trois à huit atomes et représenté soit par la formule (IVₐ), soit par la formule (IV_{b}), on désigne plus particulièrement le 1,2-propanediol, le 1,2-butanediol, le 1,3-butanediol, le 1,2-pentanediol, le 1,2-hexanediol, ou le 2-méthyl 2,4-pentanediol.

Les exemples suivants illustrent l'invention sans toutefois la limiter.

### Préparations de compositions (C₁) selon l'invention

### Le mode opératoire communs est le suivant :

➢ Introduction, à température ambiante et sous agitation mécanique, de 20% massique de Glycocolle (Glycine) dans 80% massique d'un mélange Eau/diol (85/15 massique),
➢ Ajout de soude 30% pour atteindre pH ~ 10,
➢ Coulée progressive de chlorure d'octanoyle (0,9 équivalent molaire) en maintenant le pH à -10 à l'aide de soude 30%, avec une température de 18 à 36°C.
➢ Cassage à l'acide chlorhydrique 37% qsp pH~2,
➢ Soutirage des eaux mères à 60°C,
➢ Lavage à l'eau saumurée 5%,
➢ Décantation et soutirage à 65°C.

Les caractéristiques des compositions (C₁) sont consignées dans le tableau 1 suivant :

### Préparations d'une composition (C₂₁) comparative

» Introduction, à température ambiante et sous agitation mécanique, de 20% massique de Glycocolle (Glycine) dans 80% massique d'un mélange Eau/diol (85/15 massique),
» Ajout de soude 30% pour atteindre pH ~ 10,
➢ Coulée progressive de chlorure de lauroyle (0,9 équivalent molaire) en maintenant le pH à -10 à l'aide de soude 30%, avec une température de 18 à 36°C.
➢ Cassage à l'acide chlorhydrique 37% qsp pH~2,
➢ Soutirage des eaux mères à 60°C,
➢ Lavage à l'eau saumurée 5%,
➢ Décantation et soutirage à 65°C.

Les caractéristiques de la composition (C₂₁) ainsi obtenue, sont consignées dans le tableau 2 ci-après.

### Préparations d'une composition (C₂₂) comparative

➢ Introduction, à température ambiante et sous agitation mécanique, de 20% massique d'un mélange de glycine, d'acide L-aspartique, d'acide L-glutamique et de L-alanine, dans les proportions massiques Glycine/acide L-aspartique/acide L-glutamique/L-alanine, de 10%/35%/45%/10% pour 100% massique dudit mélange d'acides aminés, dans 80% massique d'un mélange Eau/diol (85/15 massique),
➢ Ajout de soude 30% pour atteindre pH ~ 10,
➢ Coulée progressive de chlorure de lauroyle (0,9 équivalent molaire) en maintenant le pH à ∼10 à l'aide de soude 30%, avec une température de 18 à 36°C.
➢ Cassage à l'acide chlorhydrique 37% qsp pH~2,
➢ Soutirage des eaux mères à 60°C,
➢ Lavage à l'eau saumurée 5%,
➢ Décantation et soutirage à 65°C.
➢ Les caractéristiques de la composition (C₂₂) ainsi obtenue, sont consignées dans le tableau 2 ci-après.

**Table1**

| | **(C_{1A})** | **(C_{1B})** | (C_{1C}) |
|---|---|---|---|
| Diol (IVₐ) ou (IV_{b}) | **2-méthyl pentane-2,4 diol** | **1,2-propanediol** | 1,3-propanediol |
| Teneur en diol (IVₐ) ou (IV_{b}) | 8,9% en masse | 1,2% en masse | 1,1% en masse |
| Teneur en acide octanoïque (V) | **0,8%** en masse | **2,2%** en masse | 3,9% en masse |
| Teneur en eau | 19,0% en masse | 19,8% en masse | 16,0% en masse |
| Teneur en N-octanoyl glycine (I) | 71,3% en masse | 76,8% en masse | 78,5% en masse |
| Présence de sous-produits | Non | Non | 0,5% en masse |
| pH de la composition | pH = 1,8 | pH = 2,0 | pH = 1,9 |

| | **(C_{1D})** | (C_{1E}) | (C_{1F}) |
|---|---|---|---|
| Diol (IVₐ) ou (IV_{b}) | **1,2-butanediol** | **1,3-butanediol** | 1,4-butanediol |
| Teneur en diol (IVₐ) ou (IV_{b}) | 8,1% en masse | 3,8% en masse | 0,9% en masse |
| Teneur en acide octanoïque (V) | **0,6%** en masse | 2,4% en masse | 3,0% en masse |
| Teneur en eau | 19,1% en masse | 20,2% en masse | 23,0% en masse |
| Teneur en N-octanoyl glycine (I) | 72,2% en masse | 73,6% en masse | 71,9% en masse |
| Présence de sous-produits | Non | Non | 1,2% en masse |
| pH de la composition | pH = 1,6 | pH = 1,5 | pH = 1,7 |

| | **(C_{1G})** | (C_{1H}) | **(C_{1I})** |
|---|---|---|---|
| Diol (IVₐ) ou (IV_{b}) | **1,2-pentanediol** | 1,5-pentanediol | **1,2-hexanediol** |
| Teneur en diol (IVₐ) ou (IV_{b}) | 6,6% en masse | 2,2% en masse | 10,1% en masse |
| Teneur en acide octanoïque (V) | 0,8% en masse | 2,9% en masse | 0,6% en masse |
| Teneur en eau | 19,0% en masse | 18,5% en masse | 16,2% en masse |
| Teneur en N-octanoyl glycine (I) | 73,6% en masse | 74,6% en masse | 73,1% en masse |
| Présence de sous-produits | Non | 1,8% en masse | Non |
| pH de la composition | pH = 1,8 | pH = 2,1 | pH = 2,2 |

**Table 2**

| | (C21) | (C22) |
|---|---|---|
| Diol (IVₐ) ou (IV_{b}) | 1,2-propanediol | 1,2-propanediol |
| Teneur en diol (IVₐ) ou (IV_{b}) | 0,9% en masse | 1,9% en masse |
| Teneur en acide laurique (V) | 4,1% en masse | 12,9% en masse |
| Teneur en eau | 11,0% en masse | 27,5% en masse |
| Teneur en N-lauroyl glycine (I) | 83,2 % en masse | 11,9 % en masse |
| Teneur en N-lauroyl alanine (I) | - | 11,0 % en masse |
| Teneur en N-lauroyl acide aspartique (I) | - | 12,2 % en masse |
| Teneur en N-lauroyl acide glutamique (I) | - | 20,4 % en masse |
| Présence de sous-produits | 0,8% en masse | 2,2% en masse |
| pH de la composition | pH = 2,1 | pH = 2,2 |

L'analyse des compositions (C_{1A}), (C_{1B}) et (C_{1D}), (C_{1E}) et (C_{1G}) , objet de la présente invention fait apparaître qu'elles ne contiennent pas de sous-produits contrairement aux compositions (C_{1C}), (C_{1F}), (C₂₁) et (C₂₂), et que leur concentration en acide gras est inférieure à celles des compositions (C_{1C}), (C_{1H}), (C_{1F}), (C₂₁) et (C₂₂).

## Revendications

1. Procédé de préparation de la composition (C₁) comprenant, pour 100% de sa masse :
(a) - Une proportion massique supérieure ou égale à 50% en masse et inférieure ou égal à 95% en masse d'un composé de formule (I) ou d'un mélange de composés de formule (I) :
X-[CH₂]ₚ-C(=O)-Y-OH (I),
dans laquelle le radical X-[CH₂]ₚ-C(=O)- est choisi parmi les radicaux heptanoyle, octanoyle, décanoyle ou 10-undécylènoyle et Y représente, Y représente le radical divalent de formule (IIₐ₁) :
-[NH-CH(R₂)-C(=O)-]ₘ- (IIₐ₁),
dans laquelle m représente un nombre entier supérieur ou égal à un et inférieur ou égal à quatre et R₂ représente un atome d'hydrogène ou un radical choisi parmi les radicaux méthyle, isopropyle, isobutyle, 1-méthyl propyle, benzyle ou 3-amino propyle ;
(b) - Une proportion massique supérieure à 0% en masse et inférieure ou égale à 15% en masse d'un diol comportant de trois à huit atomes et représenté soit par la formule (IVₐ) :
R^{a}₁-C(R^{b}₁)(OH)-C(OH)(R^{c}₁)(R^{d}₁) (IVₐ),
dans laquelle chacun des radicaux R^{a}₁, R^{b}₁, R^{c}₁ et R^{d}₁ représentent indépendamment les uns des autres, un atome d'hydrogène ou radical aliphatique saturé comportant de un à cinq atomes de carbone, soit par la formule (IV_{b}) :
R^{a}₁-C(R^{b}₁)(OH)-[C(R^{e}₁)(R^{f}₁)]ₜ-C(OH)(R^{c}₁)(R^{d}₁) (IV_{b}),
dans laquelle t est égal à un, deux ou trois, chacun des radicaux R^{a}₁, R^{b}₁, R^{c}₁, R^{d}₁, R^{e}₁ et R^{f}₁ représentent indépendamment les uns des autres, un atome d'hydrogène ou radical aliphatique saturé comportant de un à cinq atomes de carbone, étant entendu que l'un au moins des radicaux R^{a}₁ ou R^{b}₁ et/ou l'un au moins des radicaux R^{c}₁ ou R^{d}₁ ne représentent pas un atome d'hydrogène, ledit diol de formule (Iva) ou (IVb) étant choisi parmi le 1,2-propanediol, 1,2-butanediol, le 1,3-butanediol, le 1,2-pentanediol, le 1,2-hexanediol, le 1,2-octanediol, le 2,3-butanediol, le 2,3-pentanediol, le 2,3-hexanediol, le 2,5-hexanediol ou le 2-méthyl 2,4-pentanediol.
(c) - Une proportion massique supérieure ou égale à 0% en masse et inférieure ou égale à 5% en masse d'un composé de formule (V) ou d'un mélange de composés de formule (V) :
X-[CH₂]ₚ-C(=O)-OH (V),
dans laquelle le radical X-[CH₂]ₚ-C(=O)- est choisi parmi les radicaux heptanoyle, octanoyle, décanoyle ou 10-undécylènoyle, ou d'un mélange de dits composés de formule (V) ; et
(d) - Une proportion massique supérieure à 0% en masse et inférieure à 50% en masse d'eau,
étant entendu que le pH de ladite composition est inférieur ou égal à 3,
**caractérisé en ce qu'**il comprend les étapes successives suivantes :
- Une étape a) au cours de laquelle un ledit diol comportant de trois à huit atomes et représenté soit par la formule (IVₐ), soit par la formule (IV_{b}) et choisi parmi le 1,2-propanediol, 1,2-butanediol, le 1,3-butanediol, le 1,2-pentanediol, le 1,2-hexanediol, le 1,2-octanediol, le 2,3-butanediol, le 2,3-pentanediol, le 2,3-hexanediol, le 2,5-hexanediol ou le 2-méthyl 2,4-pentanediol, est mélangé avec de l'eau, pour former un mélange (S₁) comportant pour 100% de sa masse, de 5% en masse à 70% en masse dudit diol de formule (IVₐ) ou de formule (IV_{b}) et de 30% en masse à 95% en masse d'eau ;
- Une étape b) au cours de laquelle un composé de formule (VIₐ) : 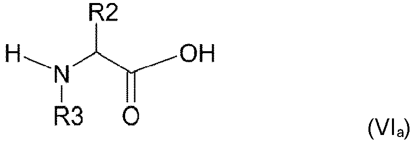 dans laquelle le radical R₃ représente un atome d'hydrogène et R₂ est tel que défini dans la formule (I), est ajouté au dit mélange (S₁) en une proportion telle que le mélange (M₁) obtenu comporte pour 100% de sa masse de 5% en masse à 50% en masse dudit composé de formule (VIₐ) ou (VI_{b}) ou dudit mélange de composés de formule (VIₐ) et/ou (VI_{b}) et de 50% en masse à 95% en masse dudit mélange (S₁) ;
- Une étape c) au cours de laquelle ledit mélange (M₁) est additionné de soude ou de potasse pour former un mélange (M₂) ayant un pH supérieur ou égal à 9 ;
- Une étape d) au cours de laquelle un chlorure d'acide de formule (VII) :
X-[CH₂]_{b}-C(=O)-Cl (VII),
dans laquelle le radical X-[CH₂]ₚ-C(=O)- est tel que défini dans la formule (I) précédente, ou un mélange de chlorures d'acide de formule (VII), est versé dans ledit mélange (M₂), en maintenant le pH à une valeur supérieure ou égale à 9 par addition conjointe de soude ou de potasse, ladite étape e) résultant en la formation d'un mélange (M₃) ;
- Une étape e) au cours de laquelle ledit mélange (M₃) est acidifié jusqu'à atteindre une valeur de pH inférieure ou égale à 3 ;
- Une étape f) de décantation au fin de soutirage des eaux-mères ;
- si nécessaire ou si désiré, au moins une étape g) de lavage à l'eau saumurée pour obtenir après décantation, ladite composition recherchée.

2. Procédé selon la revendication 1, de préparation de la composition (C₁) pour laquelle, dans la formule (I), X-[CH₂]ₚ-C(=O)- est choisi parmi les radicaux heptanoyle, octanoyle ou décanoyle et Y représente le radical divalent de formule (IIₐ₁) :
-[NH-CH(R₂)-C(=O)-]ₘ- (IIₐ₁),
dans laquelle m représente un nombre entier supérieur ou égal à un et inférieur ou égal à quatre et R₂ représente un atome d'hydrogène ou un radical choisi parmi les radicaux méthyle, isopropyle, isobutyle, 1-méthyl propyle, benzyle ou 3-amino propyle.

3. Procédé selon la revendication 2, de préparation de la composition (C₁) pour laquelle, dans la formule (I), le radical divalent Y représente le radical divalent de formule (II'ₐ₁) :
-NH-CH₂-C(=O)- (II'ₐ₁),

4. Procédé selon la revendication 3, de préparation de la composition (C₁) pour laquelle, dans les formules (I) et (V), le radical monovalent X-(CH₂)ₚ-C(=O)- représente le radical octanoyle.

5. Procédé selon la revendication 1, de préparation de la composition (C₁) pour laquelle, dans les formules (I) et (V), le radical monovalent X-(CH₂)ₚ-C(=O)- représente le radical 10-undécylénoyle et Y représente le radical divalent de formule (IIₐ₂) :
-[NH-CH(benzyle)-C(=O)-]ₘ- (IIₐ₂),

6. Procédé selon l'une quelconque des revendication 1 à 5, de préparation de la composition (C₁) pour laquelle, ledit diol comportant de trois à huit atomes représenté soit par la formule (IVₐ), soit par la formule (IV_{b}), est choisi parmi le 1,2-propanediol, le 1,2-butanediol, le 1,3-butanediol, le 1,2-pentanediol, le 1,2-hexanediol ou le 2-méthyl 2,4-pentanediol.

## Patentansprüche

1. Verfahren zur Herstellung der Zusammensetzung (C₁), umfassend auf 100 % ihrer Masse:
(a) - einen Massenanteil größer oder gleich 50 Massen-% und kleiner oder gleich 95 Massen-% einer Verbindung der Formel (I) oder eines Gemischs von Verbindungen der Formel (I):
X-[CH₂]ₚ-C(=O)-Y-OH (I),
in der der Rest X-[CH₂]ₚ-C(=O)- aus Heptanoyl-, Octanoyl- , Decanoyl- oder 10-Undecylenoylresten ausgewählt ist und Y für den zweiwertigen Rest der Formel (IIₐ₁) steht:
-[NH-CH(R₂)-C(=O)-]ₘ- (IIₐ₁),
in der m für eine ganze Zahl größer als oder gleich eins und kleiner als oder gleich vier steht und R₂ für ein Wasserstoffatom oder einen Rest, der aus Methyl-, Isopropyl-, Isobutyl-, 1-Methylpropyl-, Benzyl- und 3-Aminopropylresten ausgewählt ist, steht;
(b) - einen Massenanteil größer als 0 Massen-% und kleiner als oder gleich 15 Massen-% eines Diols mit drei bis acht Atomen, das entweder durch die Formel (IVₐ) wiedergegeben wird:
R^{a}₁-C(R^{b}₁)(OH)-C(OH)(R^{c}₁)(R^{d}₁) (IVₐ),
in der jeder der Reste R^{a}₁, R^{b}₁, R^{c}₁ und R^{d}₁ unabhängig von den anderen für ein Wasserstoffatom oder einen gesättigten aliphatischen Rest mit eins bis fünf Kohlenstoffatomen steht, oder durch die Formel (IV_{b}):
R^{a}₁-C (R^{b}₁)(OH)-[C(R^{e}₁)(R^{f}₁)]ₜ-C(OH)(R^{c}₁)(R^{d}₁) (IV_{b}),
in der t gleich eins, zwei oder drei ist, jeder der Reste R^{a}₁, R^{b}₁, R^{c}₁, R^{d}₁, R^{e}₁ und R^{f}₁ unabhängig von den anderen für ein Wasserstoffatom oder einen gesättigten aliphatischen Rest mit eins bis fünf Kohlenstoffatomen steht, wobei es sich versteht, dass mindestens einer der Reste R^{a}₁ oder R^{b}₁ und/oder mindestens einer der Reste R^{c}₁ oder R^{d}₁ nicht für ein Wasserstoffatom stehen, wobei das Diol der Formel (IVa) oder (IVb) aus 1,2-Propandiol, 1,2-Butandiol, 1,3-Butandiol, 1,2-Pentandiol, 1,2-Hexandiol, 1,2-Octandiol, 2,3-Butandiol, 2,3-Pentandiol, 2,3-Hexandiol, 2,5-Hexandiol oder 2-Methyl-2,4-pentandiol ausgewählt ist;
(c) - einen Massenanteil größer als oder gleich 0 Massen-% und kleiner als oder gleich 5 Massen-% einer Verbindung der Formel (V) oder eines Gemischs von Verbindungen der Formel (V):
X-[CH₂]ₚ-C(=O)-OH (V),
in der der Rest X-[CH₂]ₚ-C(=O)- aus Heptanoyl-, Octanoyl- , Decanoyl- oder 10-Undecylenoylresten ausgewählt ist, oder eines Gemischs der Verbindungen der Formel (V); und
(d) - einen Massenanteil größer als 0 Massen-% und kleiner als 50 Massen-% Wasser,
wobei es sich versteht, dass der pH-Wert der Zusammensetzung kleiner als oder gleich 3 ist, **dadurch gekennzeichnet, dass** es die folgenden aufeinanderfolgenden Schritte umfasst:
- einen Schritt a), bei dem man das Diol mit drei bis acht Atomen, das entweder durch die Formel (IVₐ) oder durch Formel (IV_{b}) wiedergegeben wird und aus 1,2-Propandiol, 1,2-Butandiol, 1,3-Butandiol, 1,2-Pentandiol, 1,2-Hexandiol, 1,2-Octandiol, 2,3-Butandiol, 2,3-Pentandiol, 2,3-Hexandiol, 2,5-Hexandiol oder 2-Methyl-2,4-pentandiol ausgewählt ist, mit Wasser mischt, um eine Mischung (S₁) zu bilden, die auf 100 % ihrer Masse 5 Massen-% bis 70 Massen-% des Diols der Formel (IVₐ) oder der Formel (IV_{b}) und 30 Massen-% bis 95 Massen-% Wasser umfasst;
- einen Schritt b), bei dem man eine Verbindung der Formel (VIₐ): 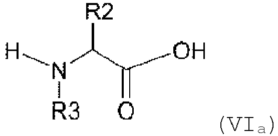 in der der Rest R₃ für ein Wasserstoffatom steht und R₂ wie in der Formel (I) definiert ist, in einem solchen Anteil zu der Mischung (S₁) gibt, dass die erhaltene Mischung (M₁) auf 100 % ihrer Masse 5 Massen-% bis 50 Massen-% der Verbindung der Formel (VIₐ) oder (VI_{b}) oder des Gemischs von Verbindungen der Formel (VIₐ) und/oder (VI_{b}) und 50 Massen-% bis 95 Massen-% der Mischung (S₁) umfasst;
- einen Schritt c), bei dem man die Mischung (M₁) mit Natriumhydroxid oder Kaliumhydroxid versetzt, um eine Mischung (M₂) mit einem pH-Wert größer als oder gleich 9 zu bilden;
- einen Schritt d), bei dem man ein Säurechlorid der Formel (VII):
X-[CH₂]ₚ-C(=O)-CI (VII),
in der der Rest X-[CH₂]ₚ-C(=O)- wie in der vorhergehenden Formel (I) definiert ist, oder ein Gemisch von Säurechloriden der Formel (VII) in die Mischung (M₂) gießt, wobei der pH-Wert durch gemeinsame Zugabe von Natriumhydroxid oder Kaliumhydroxid bei einem Wert größer als oder gleich 9 erhalten wird, wobei der Schritt e) zur Bildung einer Mischung (M₃) führt;
- einen Schritt e), bei dem man die Mischung (M₃) bis zum Erreichen eines pH-Werts kleiner als oder gleich 3 ansäuert;
- einen Schritt f) des Dekantierens am Ende des Abziehens der Mutterlaugen;
- falls erforderlich oder falls gewünscht, mindestens einen Schritt g) des Waschens mit Salzwasser, was nach Dekantieren die gewünschte Zusammensetzung ergibt.

2. Verfahren nach Anspruch 1 zur Herstellung der Zusammensetzung (C₁), für die in der Formel (I) X-[CH₂]ₚ-C(=O)- aus Heptanoyl-, Octanoyl- oder Decanoylresten ausgewählt ist und Y für den zweiwertigen Rest der Formel (IIₐ₁) steht:
-[NH-CH(R₂)-C(=O)-]ₘ- (IIₐ₁),
in der m für eine ganze Zahl größer als oder gleich eins und kleiner als oder gleich vier steht und R₂ für ein Wasserstoffatom oder einen Rest, der aus Methyl-, Isopropyl-, Isobutyl-, 1-Methylpropyl-, Benzyl- und 3-Aminopropylresten ausgewählt ist, steht.

3. Verfahren nach Anspruch 2 zur Herstellung der Zusammensetzung (C₁), für die in der Formel (I) der zweiwertige Rest Y für den zweiwertigen Rest der Formel (II'ₐ₁) steht:
-NH-CH₂-C(=O)- (II'ₐ₁) .

4. Verfahren nach Anspruch 3 zur Herstellung der Zusammensetzung (C₁), für die in den Formeln (I) und (V) der einwertige Rest X-(CH₂)ₚ-C(=O)- für den Octanoylrest steht.

5. Verfahren nach Anspruch 1 zur Herstellung der Zusammensetzung (C₁), für die in den Formeln (I) und (V) der einwertige Rest X-(CH₂)ₚ-C(=O)- für den 10-Undecylenoylrest steht und Y für den zweiwertigen Rest der Formel (IIₐ₂) steht:
-[NH-CH(Benzyl)-C(=O)-]ₘ- (IIₐ₂).

6. Verfahren nach einem der Ansprüche 1 bis 5 zur Herstellung der Zusammensetzung (C₁), für die das Diol mit drei bis acht Atomen, das entweder durch die Formel (IVₐ) oder durch die Formel (IV_{b}) wiedergegeben wird, aus 1,2-Propandiol, 1,2-Butandiol, 1,3-Butandiol, 1,2-Pentandiol, 1,2-Hexandiol oder 2-Methyl-2,4-pentandiol ausgewählt ist.

## Claims

1. Process for the preparation of the composition (C₁) comprising, per 100% of its weight:
(a) - a proportion by weight of greater than or equal to 50% by weight and of less than or equal to 95% by weight of a compound of formula (I) or of a mixture of compounds of formula (I):
X-[CH₂]ₚ-C(=O)-Y-OH (I),
in which the X-[CH₂]ₚ-C(=O)- radical is chosen from the heptanoyl, octanoyl, decanoyl or 10-undecylenoyl radicals and Y represents the divalent radical of formula (IIₐ₁):
-[NH-CH(R₂)-C(=O)-]ₘ- (IIₐ₁),
in which m represents an integer greater than or equal to one and less than or equal to four and R₂ represents a hydrogen atom or a radical chosen from the methyl, isopropyl, isobutyl, 1-methylpropyl, benzyl or 3-aminopropyl radicals;
(b) - a proportion by weight of greater than 0% by weight and of less than or equal to 15% by weight of a diol comprising from three to eight atoms and represented either by the formula (IVₐ):
R^{a}₁-C(R^{b}₁)(OH)-C(OH)(R^{c}₁)(R^{d}₁) (IVₐ),
in which each of the R^{a}₁, R^{b}₁, R^{c}₁ and R^{d}₁ radicals represent, independently of one another, a hydrogen atom or a saturated aliphatic radical comprising from one to five carbon atoms, or by the formula (IV_{b}):
R^{a}₁-C (R^{b}₁)(OH)-[C(R^{e}₁)(R^{f}₁)]ₜ-C(OH)(R^{c}₁)(R^{d}₁) (IV_{b}),
in which t is equal to one, two or three, each of the R^{a}₁, R^{b}₁, R^{c}₁, R^{d}₁, R^{e}₁ and R^{f}₁ radicals represent, independently of one another, a hydrogen atom or a saturated aliphatic radical comprising from one to five carbon atoms, it being understood that one at least of the R^{a}₁ or R^{b}₁ radicals and/or one at least of the R^{c}₁ or R^{d}₁ radicals do not represent a hydrogen atom, said diol of formula (IVₐ) or (IV_{b}) being chosen from 1,2-propanediol, 1,2-butanediol, 1,3-butanediol, 1,2-pentanediol, 1,2-hexanediol, 1,2-octanediol, 2,3-butanediol, 2,3-pentanediol, 2,3-hexanediol, 2,5-hexanediol or 2-methyl-2,4-pentanediol,
(c) - a proportion by weight of greater than or equal to 0% by weight and of less than or equal to 5% by weight of a compound of formula (V) or of a mixture of compounds of formula (V):
X-[CH₂]ₚ-C(=O)-OH (V),
in which the X-[CH₂]ₚ-C(=O)- radical is chosen from the heptanoyl, octanoyl, decanoyl or 10-undecylenoyl radicals, or of a mixture of said compounds of formula (V): and
(d) - a proportion by weight of greater than 0% by weight and of less than 50% by weight of water,
it being understood that the pH of said composition is less than or equal to 3, **characterized in that** it comprises the following successive stages:
- a stage a) during which said diol comprising from three to eight atoms and represented either by the formula (IVₐ) or by the formula (IV_{b}) and chosen from 1,2-propanediol, 1,2-butanediol, 1,3-butanediol, 1,2-pentanediol, 1,2-hexanediol, 1,2-octanediol, 2,3-butanediol, 2,3-pentanediol, 2,3-hexanediol, 2,5-hexanediol or 2-methyl- 2,4-pentanediol is mixed with water, to form a mixture (S₁) comprising, per 100% of its weight, from 5% by weight to 70% by weight of said diol of formula (IVₐ) or of formula (IV_{b}) and from 30% by weight to 95% by weight of water;
- a stage b) during which a compound of formula (VIₐ): 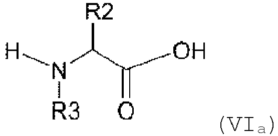 in which the R₃ radical represents a hydrogen atom and R₂ is as defined in the formula (I), is added to said mixture (S₁) in a proportion such that the mixture (M₁) obtained comprises, per 100% of its weight, from 5% by weight to 50% by weight of said compound of formula (VIₐ) or (VI_{b}) or of said mixture of compounds of formula (VIₐ) and/or (VI_{b}) and from 50% by weight to 95% by weight of said mixture (S₁) ;
- a stage c) during which sodium hydroxide or potassium hydroxide is added to said mixture (M₁) to form a mixture (M₂) having a pH of greater than or equal to 9;
- a stage d) during which an acid chloride of formula (VII) :
X-[CH₂]ₚ-C(=O)-Cl (VII),
in which the X-[CH₂]ₚ-C(=O)- radical is as defined in the preceding formula (I), or a mixture of acid chlorides of formula (VII) is poured into said mixture (M₂), while maintaining the pH at a value of greater than or equal to 9 by joint addition of sodium hydroxide or potassium hydroxide, said stage e) resulting in the formation of a mixture (M₃);
- a stage e) during which said mixture (M₃) is acidified until a pH value of less than or equal to 3 is obtained;
- a stage f) of separation by settling for the purpose of withdrawing the mother liquors;
- if necessary or if desired, a least one stage g) of washing with brine in order to obtain, after separation by settling, said sought-after composition.

2. Process according to Claim 1, of preparation of the composition (C₁) for which, in the formula (I), X-[CH₂]ₚ-C(=O)- is chosen from the heptanoyl, octanoyl or decanoyl radicals and Y represents the divalent radical of formula (IIₐ₁):
-[NH-CH(R₂)-C(=O)-]ₘ- (IIₐ₁),
in which m represents an integer greater than or equal to one and less than or equal to four and R₂ represents a hydrogen atom or a radical chosen from the methyl, isopropyl, isobutyl, 1-methylpropyl, benzyl or 3-aminopropyl radicals.

3. Process according to Claim 2, of preparation of the composition (C₁) for which, in the formula (I), the divalent radical Y represents the divalent radical of formula (II'ₐ₁):
-NH-CH₂-C(=O)- (II'ₐ₁).

4. Process according to Claim 3, of preparation of the composition (C₁) for which, in the formulae (I) and (V), the monovalent radical X-(CH₂)ₚ-C(=O)- represents the octanoyl radical.

5. Process according to Claim 1, of preparation of the composition (C₁) for which, in the formulae (I) and (V), the monovalent radical X-[CH₂]ₚ-C(=O)- represents the 10-undecylenoyl radical and Y represents the divalent radical of formula (IIₐ₂):
-[NH-CH(benzyl)-C(=O)-]ₘ- (IIa2).

6. Process according to any one of Claims 1 to 5, of preparation of the composition (C₁) for which said diol comprising from three to eight atoms, represented either by the formula (IVₐ) or by the formula (IV_{b}), is chosen from 1,2-propanediol, 1,2-butanediol, 1,3-butanediol, 1,2-pentanediol, 1,2-hexanediol or 2-methyl-2,4-pentanediol.
